# FASCICULE DE BREVET EUROPEEN

(11) **EP 1 307 251 B1**
(45) Date de publication et mention de la délivrance du brevet: **27.10.2004**
(21) Numéro de dépôt: 01958195.8
(22) Date de dépôt: 27.07.2001
(51) Int. Cl.: A61M 5/20, A61M 5/303

(54) **SERINGUE SANS AIGUILLE FONCTIONNANT PAR MISE EN COMPRESSION DU RESERVOIR CONTENANT LE PRINCIPE ACTIF LIQUIDE**
NADELLOSE SPRITZE MIT EINEM UNTER DRUCK GESTELLTEN WIRKSTOFFBEHÄLTER
NEEDLELESS SYRINGE OPERATING BY COMPRESSION OF THE RESERVOIR CONTAINING THE ACTIVE LIQUID

(30) Priorité: 28.07.2000 FR 0009963
(43) Date de publication de la demande: 07.05.2003
(73) Titulaire: CROSSJECT, 75004 Paris (FR)
(72) Inventeur: ALEXANDRE, Patrick, F-70100 Gray (FR); BROUQUIERES, Bernard, F-83100 Toulon (FR); PECH, Bernard, F-75004 Paris (FR)
(74) Mandataire: Waligorski, Carol
(86) Numéro de dépôt international: PCT/FR2001/002477
(87) Numéro de publication internationale: WO 2002/009796

(56) Documents cités:
- US-A- 2 699 166
- US-A- 5 730 723

## Description

Le domaine technique de l'invention est celui des seringues sans aiguille préremplies et jetables, fonctionnant avec un générateur de gaz, et utilisées pour les injections intradermiques, sous-cutanées et intramusculaires, de principe actif liquide à usage thérapeutique en médecine humaine ou vétérinaire.

Plus particulièrement l'invention concerne un dispositif d'injection cutanée comprenant un corps, et destiné à injecter un principe actif liquide contenu dans un réservoir tubulaire constitué par un tube fermé à son extrémité amont par un piston de refoulement et comportant à son extrémité aval un ensemble d'injection, ce réservoir étant logé dans le corps du dispositif d 'injection qui comporte en amont du piston un générateur de gaz à haute pression permettant d'exercer une pression sur ce piston de refoulement.

Pour les dispositifs d'injection selon l'invention, un principe actif liquide est constitué par un liquide plus ou moins visqueux, ou un mélange de liquide, ou un gel. Le principe actif peut être un solide mis en solution dans un solvant approprié pour l'injection. Il peut également être représenté par un solide pulvérulent mis en suspension plus ou moins concentré dans un liquide approprié. La granulométrie du principe doit être compatible avec le diamètre des conduits pour éviter les bouchages.

Pour les seringues préremplies, les impératifs liés, d'une part, à la compatibilité à long terme entre le principe actif liquide et le réservoir qui le contient, et, d'autre part, au contrôle réglementaire du remplissage dudit réservoir lors du procédé de préremplissage, conduisent à la réalisation de réservoir essentiellement transparent dont le matériau constitutif est compatible avec le principe actif à injecter.

Les réservoirs en verre répondent à cette double exigence. Seulement, il apparaît rapidement une limitation à l'usage de ce type de réservoir, due essentiellement au fait que le principe actif liquide est expulsé du réservoir par la poussée d'au moins un piston solide coulissant dans ledit réservoir, induisant alors une pression interne très élevée pouvant conduire à l'éclatement dudit réservoir. Un moyen connu de se prémunir contre ce genre d'événement indésirable correspond à l'utilisation d'une gaine enveloppante de compression du réservoir apte à contrecarrer la pression interne générée par le piston et à préserver l'intégrité du tube en verre durant toute la durée de l'injection.

Des dispositifs de compression du réservoir en verre dans des seringues sans aiguille destinées à injecter du principe actif liquide existent déjà et ont fait l'objet de plusieurs brevets. On peut citer en particulier le brevet EP 0 792 174 décrivant un flacon en verre conçu pour être utiliser comme capsule dans un injecteur sans aiguille, ledit flacon étant mis dans un état de compression permanent au moyen d'une gaine enveloppante pouvant, par exemple, être en polycarbonate. Pour ce type de réalisation deux inconvénients apparaissent : d'une part, le procédé de fabrication nécessite une étape supplémentaire consistant à insérer la gaine autour du flacon et, d'autre part, le flacon subit inutilement un effort permanent de compression, y compris en phase de stockage.

Le brevet US 2 764 977 décrit un dispositif d'injection hypodermique faisant intervenir un réservoir pouvant être en verre et placé dans une gaine en caoutchouc. La mise en compression du réservoir par la gaine n'intervient qu'au moment du fonctionnement du dispositif et est provoquée par le passage d'un piston solide de poussée à travers le réservoir pour expulser le principe actif liquide. En effet, la longueur de la gaine étant légèrement supérieure à celle du réservoir, le piston, lorsqu'il amorce son déplacement, induit une pression sur le bord de la gaine débordant du tube, cette pression se transmettant à l'ensemble de la gaine qui réagit en comprimant le réservoir. Ce type de dispositif nécessite une grande homogénéité au niveau du dimensionnement et de la géométrie des pièces interagissant entre elles, de façon à obtenir une parfaite équipartition des efforts s'exerçant sur la périphérie du réservoir. Enfin, le brevet US 2 699 166 (état de la technique le plus proche) divulgue également un dispositif d'injection hypodermique impliquant un réservoir entouré d'une gaine en caoutchouc. La compression du réservoir par la gaine ne se produit que lors du fonctionnement du dispositif, durant lequel un piston se déplace, provoquant, dans une première phase, l'arrivée d'un liquide inerte incompressible dans un espace situé autour de la gaine de façon à comprimer ladite gaine autour du réservoir, puis, dans une deuxième phase, l'expulsion du principe actif liquide en poussant ledit principe dans le réservoir. Ce type de dispositif est rendu complexe par la présence d'un circuit hydraulique parallèle et est alourdi significativement par le liquide incompressible dudit circuit. De plus, le circuit hydraulique ne devant pas interférer directement sur l'expulsion du principe actif, il est nécessaire de recourir à un mécanisme de fonctionnement impliquant des pièces de géométrie particulière nécessitant un usinage adapté et donc un coût plus élevé.

La seringue sans aiguille selon l'invention dispose de moyens de compression pneumatique du réservoir contenant le principe actif liquide permettant de s'affranchir de tous les problèmes précités mis en relief par l'état de la technique. En effet, ces moyens ne sont activés que lors du fonctionnement desdites seringues, ils n'exercent donc aucune contrainte sur ledit réservoir en mode stockage, évitant de lui faire subir inutilement un effort permanent. De plus, ces moyens de compression n'impliquent aucune pièce solide supplémentaire spécialement fabriquée pour contribuer à la compression du réservoir, diminuant ainsi les risques de mauvais fonctionnement en multipliant les pièces impliquées. Enfin, ces moyens de compression des seringues selon l'invention sont simples, directs et efficaces ne nécessitant essentiellement que la présence de conduits reliant le générateur de gaz à un espace libre situé autour du réservoir. Il a en effet été découvert qu'il était possible de s'affranchir de toute gaine enveloppante de compression et de protection, et cela même dans les cas extrêmes lorsque le dispositif d'injection comporte un tube en verre d'épaisseur moyenne de 2 à 2,5 mm et un générateur pyrotechnique de gaz mettant en oeuvre des poudres propulsives du type des poudres de chasse ou des poudres à canon permettant l'obtention d'une pression pouvant atteindre plus de 400 bars.

L'objet de la présente invention concerne un dispositif d'injection cutanée comprenant un corps et destiné à injecter un principe actif liquide contenu dans un réservoir tubulaire constitué par un tube fermé à son extrémité amont par un piston de refoulement et comportant à son extrémité aval un ensemble d'injection, ce réservoir étant logé dans le corps du dispositif d'injection qui comporte en amont du piston un générateur de gaz à haute pression permettant d'exercer une pression sur ce piston de refoulement, caractérisé en ce qu'un espace de pressurisation par les gaz du générateur est ménagé entre le réservoir tubulaire et la paroi interne du corps.

De cette façon, le générateur de gaz exerce une double fonction :
- assurer l'expulsion du principe actif liquide par l'intermédiaire d'un piston mu par les gaz libérés,
- mettre en compression le réservoir par accumulation desdits gaz dans un espace situé autour du réservoir.

En fait, le déplacement du piston de refoulement à l'intérieur du réservoir crée instantanément une surpression interne qu'il faut compenser par une compression immédiate du réservoir. Par ce biais, le réservoir se retrouve soumis à un régime d'équipression tendant à annuler la contrainte résultante s'exerçant sur lui et éliminant tout risque de fracture. Le réservoir peut alors traverser la phase d'injection en conservant toute son intégrité malgré les très fortes sollicitations mises en jeu. Pour être pleinement efficace et assurer une bonne marge de sécurité par rapport au risque de fracture du réservoir, la compression du réservoir doit intervenir juste avant l'instauration de la pression interne engendrée par le piston qui se déplace, voire au plus tard en même temps. Cette technique reprend le principe dit « des blocs libres » rencontré dans le milieu de la Propulsion et consistant pour un bloc de propergol muni d'un canal central, à prélever, lorsqu'il est en combustion, des gaz dans ledit canal pour les réinjecter à sa périphérie, de façon à équilibrer les pressions et à éviter un éclatement prématuré dû à un accroissement de la pression interne associé à une diminution de l'épaisseur à brûler.

Cette technique est, par exemple, tout à fait adaptée aux réservoirs en verre, peu susceptibles de se déformer et réagissant plutôt suivant un mode binaire, soit en résistant aux efforts sans se modifier, soit en se cassant sans subir de phase transitoire de déformation. De plus, un réservoir en verre étant plus sensible aux efforts en traction plutôt qu'en compression, il va beaucoup mieux résister aux sollicitations internes en étant comprimé.

Selon une première variante de réalisation de l'invention, la paroi interne du corps comporte au moins trois nervures longitudinales de centrage du réservoir tubulaire et de façon préférentielle, les nervures longitudinales comportent un épaulement de calage longitudinal du réservoir tubulaire. De cette manière, le réservoir se retrouve bloqué dans la seringue à la fois selon un axe transversal grâce aux nervures longitudinales de centrage lui assurant un maintien homogène suivant son axe et selon un axe longitudinal grâce à l'épaulement de calage.

Avantageusement le tube est réalisé en verre pour répondre à la double exigence de compatibilité du principe actif avec le matériau constitutif de son réservoir et de lisibilité pour contrôler le remplissage dudit réservoir.

Préférentiellement, la surface externe du piston de refoulement, dirigée vers le générateur de gaz constitue un déflecteur des gaz vers l'espace de pressurisation, de façon à favoriser une accumulation rapide des gaz vers l'espace de pressurisation juste avant que le piston n'ait pu commencer à se mouvoir, permettant de mettre en compression le réservoir avant la génération d'une surpression interne dans ledit réservoir. De façon avantageuse, l'extrémité du piston dirigée vers le générateur de gaz a une forme conique pour optimiser l'écoulement des gaz autour du réservoir en déviant directement lesdits gaz vers l'espace de pressurisation.

Préférentiellement, le piston de refoulement s'étend au dessus du tube et prend appui sur les nervures longitudinales du corps.

De façon préférentielle, le piston comporte une amorce de rupture par cisaillement qui correspond aux dimensions internes du tube. Par ce biais, le piston joue le rôle d'un opercule calibré, en permettant d'instaurer un niveau de pression seuil en amont dudit piston, au delà duquel il va se rompre par effet d'emporte-pièce, au niveau de l'amorce de rupture par cisaillement, afin d'amorcer sa course dans le réservoir. L'amorce de rupture par cisaillement permet de retarder le départ du piston pour assurer une compression préalable du réservoir périphérique.

Avantageusement, l'amorce de rupture par cisaillement correspond à un sillon annulaire, creusé dans le piston, le diamètre dudit sillon étant approximativement égal au diamètre interne du tube.

De façon avantageuse, le générateur de gaz est un générateur pyrotechnique comprenant une charge pyrotechnique et un système d'initiation de ladite charge.

Préférentiellement, l'ensemble d'injection comprend un obturateur aval qui comporte au moins un trou borgne ménageant une paroi étanche crevable, cette paroi s'étendant entre le fond de ce trou borgne et le principe actif. De façon préférentielle, l'ensemble d'injection comprend au moins un tube d'injection qui pénètre dans un trou borgne de l'obturateur aval et peut transpercer la paroi étanche crevable située entre l'extrémité distale de ce tube d'injection et le principe actif. Avantageusement, chaque tube d'injection est solidaire du corps du dispositif.

Le principe actif liquide se retrouve emprisonné dans le tube du réservoir entre le piston amont et l'obturateur aval. Lorsque le piston pénètre dans le tube, la pression produite dans le principe actif liquide se transmet à l'obturateur aval, qui se déforme légèrement en étant repoussé vers l'extérieur du réservoir, puis finit par s'ouvrir au niveau de la paroi étanche crevable, grâce à chaque tube d'injection agissant chacun comme un poinçon rigide sur ladite paroi. En étant solidaire du corps du dispositif, les tubes d'injection sont indépendants du réservoir et ne risquent donc pas de subir une quelconque influence de la part du piston qui se déplace.

Selon une autre variante de réalisation de l'invention, l'obturateur aval est réalisé en matériau compressible, de sorte que, sous l'effet de la pression générée par le piston qui se déplace, l'obturateur s'écrase au fond du dispositif d'injection, en provoquant le percement de la paroi étanche par les tubes d'injection inamovibles.

Selon un mode de réalisation préféré de l'invention, l'obturateur aval s'étend en dessous du tube et se trouve encastré dans le corps.

De façon avantageuse, le corps comprend une semelle et un organe de compression qui est interposé entre l'obturateur aval et ladite semelle. Cet organe de compression qui peut, par exemple, être représenté par une bague élastique ou un ressort, agit comme un moyen de sécurité en ménageant un espace compressible empêchant chaque tube d'injection de crever la paroi de l'obturateur aval par fixation de la semelle sur le dispositif d'injection. Cet espace compressible sert également de dispositif d'amortissement pour minimiser le choc dû à l'impact du piston.

Préférentiellement, le piston de refoulement et l'obturateur aval sont au moins partiellement réalisés en matériau amortissant. En effet, sous l'effet des gaz produits par le générateur, le piston va amorcer un déplacement dans le réservoir qui va se traduire instantanément par une surpression interne qui est transmise à l'obturateur aval. Le déplacement s'effectuant sur une durée extrêmement brève et avec une vitesse très élevée, le risque de voir se produire des interférences d'ondes de pression dans le piston et dans l'obturateur aval pouvant conduire notamment à l'émission d'un jet liquide en saccades, est important. Aussi, dans le but d'éviter ce type d'inconvénients, il est souhaitable d'atténuer l'intensité des différentes ondes parasites en ayant recourt à des matériaux amortissants pouvant garantir une poussée du piston régulière et homogène.

De façon préférentielle, le corps comprend une partie centrale tubulaire et la semelle qui est munie d'au moins un tube d'injection est solidarisée de façon étanche à la partie centrale tubulaire dudit corps. Autrement dit, le corps du dispositif d'injection cutanée selon l'invention est constitué d'une partie centrale tubulaire et d'une semelle, et, avantageusement, la semelle est assimilable à un couvercle fileté venant se visser autour de l'extrémité filetée de ladite partie tubulaire.

De façon avantageuse, tous les tubes d'injection sont disposés sur la semelle de manière à ce que leurs axes soient orientés suivant le sens de propagation du piston dans le réservoir.

Préférentiellement, le générateur de gaz comporte des orifices d'évacuation des gaz dirigés au moins partiellement vers l'espace de pressurisation. Cette conformation particulière des orifices d'évacuation répond au souci d'instaurer rapidement une compression autour du réservoir juste avant la mise en mouvement du piston. Ces orifices permettent de disperser le flux gazeux selon une direction approximativement latérale par rapport à l'axe du réservoir évitant d'exercer une poussée prématurée sur le piston.

De façon avantageuse, un joint d'étanchéité est disposé entre la partie aval du corps et l'ensemble d'injection. Les gaz émis dans l'espace de pressurisation ne peuvent donc pas envahir un quelconque espace libre externe à l'ensemble d'injection, évitant tout risque de provoquer une contre poussée dudit ensemble d'injection pouvant contrarier l'injection.

Selon une variante de réalisation de l'invention, l'obturateur aval comporte une virole périphérique en contact avec le joint d'étanchéité. Il est également possible d'envisager un obturateur aval en caoutchouc présentant à sa périphérie un bourrelet en caoutchouc, ledit obturateur aval pouvant s'encastrer dans le dispositif, dans une position pour laquelle le bourrelet occupe une gorge annulaire creusée dans ledit dispositif et prévue à cet effet. Pour cette configuration, l'étanchéité est assurée par le bourrelet qui est écrasé dans ladite gorge. Pour une telle variante de réalisation, il est alors avantageux que l'obturateur aval comporte au moins une préperforation en correspondance avec un canal d'injection.

Enfin, l'invention porte également sur un procédé de fabrication caractérisé en ce que :
- dans une première étape, l'ensemble constitué par le réservoir tubulaire rempli du principe actif et encastré dans la semelle munie d'au moins un tube d'injection, est protégé par un embout amovible étanche, l'ensemble des opérations de cette première étape étant réalisé dans les conditions pharmaceutiques d'hygiène et de sécurité exigées pour la fabrication de médicaments,
- dans une deuxième étape, cet ensemble est fixé de façon étanche à l'extrémité aval de la partie centrale tubulaire du corps dont l'autre extrémité reçoit le générateur de gaz à haute pression, l'ensemble de ces opérations d'assemblage étant réalisé dans des conditions simples d'hygiène et de sécurité.

Les dispositifs d'injection selon l'invention mettant en jeu un dispositif de compression du réservoir de principe actif présentent l'avantage d'être économiques et peu encombrants. Ils sont peu encombrants car le dispositif de compression n'existe pas en mode de stockage et est créé en début de fonctionnement, sous forme d'un afflux gazeux ne nécessitant pas l'intervention de pièces solides supplémentaires spécialement conçues pour cette fonction de compression. Ils sont économiques car le dispositif de compression emprunte une source énergétique préexistante et ne nécessite pas d'être intégré dans un circuit de fluide parallèle et autonome. Enfin, le dispositif de compression mis en oeuvre dans les dispositifs d'injection selon l'invention est indépendant de la position et de la vitesse du piston dans le réservoir et s'avère être particulièrement efficace tout en restant simple.

On donne ci-après la description détaillée de quatre modes de réalisation préférés de l'invention en se référant aux figures 1 à 4.

La figure 1 est une vue en coupe axiale longitudinale d'un premier mode de réalisation préféré d'un dispositif d'injection selon l'invention.

La figure 2 est une vue en coupe axiale de la partie terminale d'un deuxième mode de réalisation préféré d'un dispositif d'injection selon l'invention.

La figure 3 est une vue en coupe axiale partielle d'un troisième mode de réalisation préféré d'un dispositif d'injection selon l'invention.

La figure 4 est une vue en coupe axiale de la partie terminale d'un quatrième mode de réalisation préféré d'un dispositif d'injection selon l'invention.

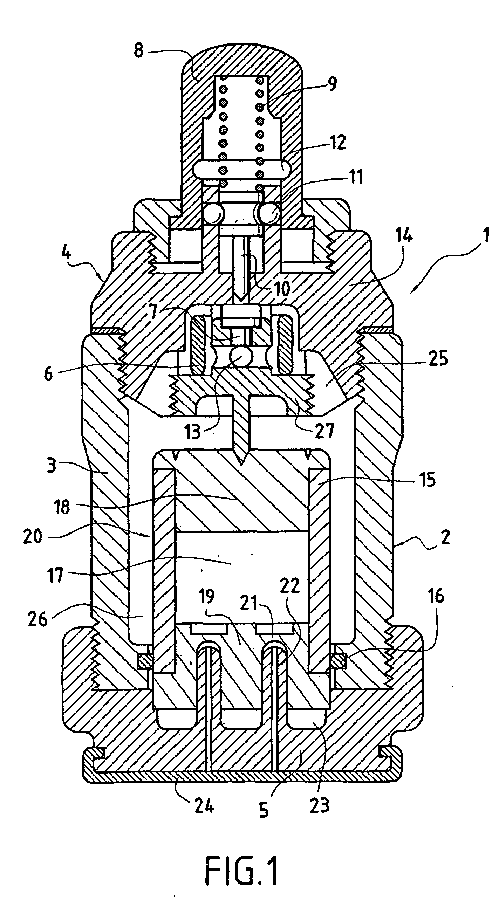

En se référant à la figure 1, un dispositif d'injection 1 selon un premier mode de réalisation préféré de l'invention est constitué par un corps 2 présentant une partie centrale tubulaire 3 ayant deux extrémités, l'une, en amont, étant destinée à recevoir un générateur de gaz pyrotechnique 4, l'autre, en aval, étant obturée par une semelle 5. Le générateur pyrotechnique 4 de gaz comprend un dispositif d'initiation de la charge pyrotechnique 6 faisant intervenir un dispositif de percussion et une amorce 7. Le dispositif de percussion, qui est déclenché par un bouton poussoir 8 comprend un ressort 9 et un percuteur 10. Le percuteur 10 est bloqué par deux billes 11 coincées entre ledit percuteur 10 et le bouton poussoir 8 et ledit bouton poussoir 8 possède une gorge interne 12 circulaire.

La charge pyrotechnique 6, qui possède un canal central, est disposée autour d'un support 27 contenant l'amorce 7, ledit support comportant dans le prolongement de ladite amorce 7 une partie creuse munie d'orifices latéraux 13. Les dimensions du support sont telles, qu'il y a étanchéité entre l'espace dans lequel est disposée la charge pyrotechnique 6 et l'espace interne du support de l'amorce 7 pouvant communiquer avec l'espace interne du bouton poussoir 8. Le percuteur 10 est représenté par un corps cylindrique dont une extrémité est pointue et l'autre extrémité est constituée par une tête plate élargie.

D'un point de vue structurel, le générateur de gaz pyrotechnique 4 comprend un corps 14 muni de trois filetages, l'un dans lequel est vissé le dispositif de percussion de la charge pyrotechnique 6, un second permettant audit corps 14 du générateur 4 d'être vissé dans la partie tubulaire 3 du corps 2 du dispositif d'injection 1, et un troisième filetage ménagé dans les nervures qui délimitent huit fentes d'évacuation 25 des gaz, ce troisième filetage permettant la fixation du support 27. Cette partie centrale tubulaire 3 a la forme d'un cylindre creux dont une extrémité est filetée sur sa surface interne pour recevoir le corps 14 du générateur pyrotechnique 4 de gaz, et l'autre extrémité a été rabattue à angle droit vers l'intérieur de ladite partie centrale 3, ménageant, au niveau de ladite extrémité, un passage dont le diamètre est inférieur au diamètre interne de la partie centrale 3. Un tube en verre 15, d'épaisseur 3 mm, dont le diamètre externe de 15 mm est sensiblement égal au diamètre dudit passage, est coincé au niveau de ce passage par l'intermédiaire d'un joint 16, une extrémité dudit tube 15 arrivant au milieu de ce passage. En continuité des orifices latéraux 13 sont situées huit fentes d'évacuation 25 présentant une courbure progressive pour rejoindre la paroi latérale interne de la partie centrale tubulaire 3 du corps 2. Ledit tube 15 qui est destiné à contenir le principe actif liquide 17 est, d'une part, obturé à sa partie amont par un piston 18 assimilable à un bouchon possédant une tête plate élargie émergeant du tube 15, ladite tête ayant un diamètre approximativement égal au diamètre externe du tube 15, et possédant sur sa surface plane externe une amorce de rupture par cisaillement sous la forme d'un sillon annulaire dont le diamètre est approximativement égal au diamètre interne du tube 15, et, d'autre part, obturé à sa partie aval par un obturateur aval 19.

L'ensemble « tube en verre 15, piston 18 et obturateur aval 19 » constitue le réservoir 20 du principe actif liquide 17. L'obturateur aval 19 est également assimilable à un bouchon possédant une tête plate élargie émergeant du tube 15, ladite tête ayant un diamètre approximativement égal au diamètre externe du tube 15 et possédant sur sa surface plane externe quatre trous borgnes. L'obturateur aval 19 possède une surface plane interne dans laquelle a été creusée une gorge annulaire de manière à ce que les quatre trous borgnes, qui sont régulièrement espacés, sont répartis sur une couronne ayant les mêmes dimensions que celle matérialisée par la gorge. L'épaisseur de l'obturateur aval 19 se retrouve donc réduite au niveau de la gorge annulaire correspondant aux trous borgnes, permettant de définir une paroi étanche crevable 21 isolant le principe actif liquide 17 des trous borgnes.

La semelle 5, qui est assimilable à un couvercle fileté, est vissée autour de la partie centrale tubulaire 3. Ladite semelle 5 possède une face plane circulaire interne comportant un évidement central cylindrique présentant un épaulement interne permettant de distinguer une partie superficielle de même diamètre que celui de la tête élargie de l'obturateur aval 19 et une partie profonde de diamètre réduit. L'obturateur aval 19 est positionné dans le dispositif d'injection de sorte qu'il obture le tube 15 et que la surface latérale externe de la tête élargie vienne en appui à la fois contre le bord replié de la partie centrale tubulaire 3 du corps 2 et la paroi latérale interne de la partie superficielle de l'évidement central cylindrique de la semelle 5, ladite paroi interne de la partie superficielle se trouvant en continuité du bord replié. La tête élargie de l'obturateur aval 19 occupe ainsi toute la partie superficielle de l'évidement si bien qu'elle vient en butée contre l'épaulement interne dudit évidement central cylindrique de la semelle 5. La partie profonde de l'évidement de la semelle 5 possède un fond plan duquel émergent quatre protubérances allongées, chacune de ces protubérances étant traversée suivant leur longueur par un canal débouchant, d'une part, à l'extrémité de ladite protubérance et, d'autre part, à l'extérieur de la semelle 5. Ces protubérances percées constituent des tubes d'injection 22. Lesdits tubes d'injection 22, qui sont solidaires de la semelle 5 et dont les axes sont parallèles à celui du tube 15 du réservoir 20, occupent les trous borgnes de l'obturateur aval 19. La partie profonde de l'évidement de la semelle 5 est constituée, entre les tubes d'injection 22, par un espace libre 23. La semelle 5 présente une face plane circulaire externe dans laquelle débouchent les canaux traversant les tubes d'injection 22, ladite face circulaire étant recouverte par un embout amovible étanche 24. Le réservoir 20 de principe actif liquide, qui est fixé au niveau de sa partie aval au corps 2 du dispositif d'injection 1, laisse apparaître un espace libre 26 situé entre sa paroi latérale externe et la paroi latérale interne de la partie centrale tubulaire 3 du corps 2 dans laquelle il est logé. Cet espace libre 26 se trouve en communication avec les huit fentes d'évacuation 25, elles-mêmes en communication avec la charge pyrotechnique 6.

Le mode de fonctionnement de ce premier mode de réalisation préféré de l'invention s'effectue comme suit.

L'utilisateur retire l'embout amovible étanche 24 qui sert de protection au dispositif d'injection 1.

Il positionne le dispositif d'injection 1 de façon à ce que la semelle 5 vienne en appui contre la peau du patient à traiter. Une pression sur le bouton poussoir 8 permet de le faire coulisser le long de la seringue jusqu'à ce que la gorge 12 vienne au niveau des billes 11 qui bloquent le percuteur 10. Un ressort 9 placé dans la seringue confère une certaine résistance au bouton poussoir 8 de façon à obliger l'utilisateur à produire un effort particulier pour enfoncer ledit bouton 8. Les billes 11 n'étant alors plus coincées, libèrent le percuteur 10 qui, sous l'effet du ressort 9 qui se détend, est propulsée vers l'amorce 7. L'amorce 7 qui est alors initiée provoque la mise à feu de la charge pyrotechnique 6 par l'intermédiaire des orifices latéraux 13. Les gaz produits par la combustion de la charge pyrotechnique 6 passent par les fentes d'évacuation 25 et envahissent instantanément l'espace de pressurisation 26 provoquant simultanément, d'une part, la compression externe du tube 15 et la compression interne de ce tube par la pression qui s'exerce sur le piston 18 réalisé en élastomère, et, d'autre part, le déplacement du réservoir 20 dans l'espace libre 23 par compression et déformation de l'obturateur aval 19 en élastomère. Une fois que la pression s'exerçant sur le piston 18 atteint une valeur seuil, ledit piston 18 cède au niveau de son amorce de rupture par cisaillement et commence à se déplacer dans le tube 15 et à exercer une poussée sur le principe actif liquide 17. L'obturateur aval 19 est alors au contact de la face plane interne de la semelle 5 et les tubes d'injection 22 agissant chacun comme un poinçon crèvent la paroi 21. Le principe actif liquide 17 envahit alors les canaux desdits tubes 22 avant d'être injecté au patient.

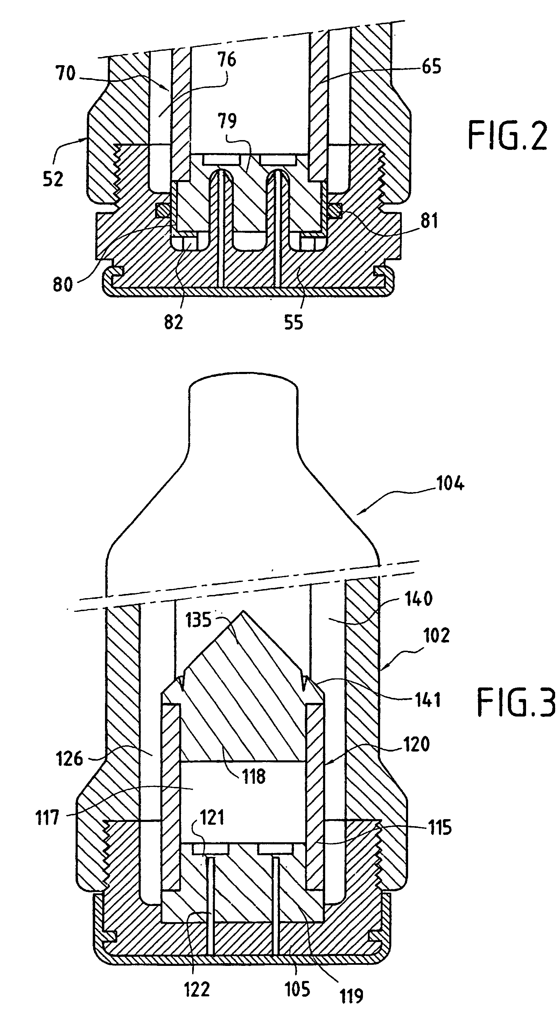

En se référant à la figure 2, un dispositif d'injection selon un deuxième mode de réalisation préféré de l'invention diffère du premier mode de réalisation préféré décrit ci avant, au niveau de la conformation de l'obturateur aval 79 et de la semelle 55 ainsi que de la fixation du réservoir 70 du corps 52 du dispositif d'injection. Une partie de la tête élargie de l'obturateur aval 79 qui est partiellement recouverte par une virole épaulée métallique 80 est insérée dans un évidement cylindrique de la semelle, l'étanchéité de ce contact étant réalisée au moyen d'un joint 81 coincé entre ladite virole 80 et la paroi latérale interne de l'évidement. Un organe de compression 82 constitué par une rondelle métallique ondulée est interposé entre la tête de l'obturateur 79 et le fond de l'évidement dans lequel elle est insérée. Ce dispositif de compression 82, qui peut revêtir la forme d'un ressort ou d'une bague compressible, permet de maintenir l'obturateur 79 dans une position donnée et sert aussi de dispositif de sécurité en garantissant un espace incompressible entre l'obturateur 79 et la semelle 55 pour éviter de crever ledit obturateur 79 tant que le générateur de gaz n'est pas déclenché. Une fois insérée dans ledit évidement, la tête élargie de l'obturateur 79 émerge dudit évidement, si bien que le tube 65 du réservoir 70 qui vient en butée contre la tête élargie se retrouve isolé du corps 52 du dispositif d'injection.

Le mode de fonctionnement d'un dispositif d'injection selon ce deuxième mode de réalisation préféré de l'invention est en tout point identique à celui décrit pour le premier mode de réalisation préféré de l'invention.

L'agencement des pièces impliquées dans ce deuxième mode de réalisation préféré de l'invention, permet d'éviter tout contact direct entre le réservoir 70 en verre et le corps solide 52 du dispositif d'injection, contact qui pourrait s'avérer préjudiciable pour ledit réservoir 70 susceptible de se briser en se frottant ou en venant s'appuyer contre une surface solide, par exemple en cas de chute du dispositif d'injection durant une manipulation ou un défaut de stokage.

En se référant à la figure 3, un dispositif d'injection selon un troisième mode de réalisation préféré de l'invention diffère du premier mode de réalisation sur les points décrits ci-après. Les gaz émis par le générateur pyrotechnique 104, dont seul le contour a été schématisé, ne sont plus diffusés transversalement dans le corps 102 du dispositif d'injection par l'intermédiaire d'orifices latéraux, mais sont produits suivant l'axe dudit corps 102 et arrivent donc sur le réservoir 120 selon une direction parallèle à son axe de révolution. Ledit réservoir 120 est fixé dans le corps 102 du dispositif, par l'intermédiaire de quatre nervures longitudinales de centrage 140 disposées sur la paroi interne dudit corps 102 en faisant un angle de 90° l'une par rapport à l'autre, lesdites nervures 140 comportant un épaulement 141 de calage longitudinal du réservoir 120. L'obturateur aval 119 qui est réalisé en matériau compressible et qui obture le tube 115 du réservoir 120 vient en appui contre le fond plan d'un évidement pratiqué dans la semelle 105 du corps 102. La tête élargie de l'obturateur 119 émerge de l'évidement et le tube 115 qui vient en butée contre ladite tête se retrouve isolé du corps 102. Le piston 118 qui obture l'extrémité amont du tube 115 émerge dudit tube 115, la partie émergeante 135 du piston 118 ayant une forme conique dont la base élargie, qui vient en appui contre le tube 115 a un diamètre supérieur au diamètre externe dudit tube 115 pour assurer un centrage avec amortissement et venir au contact de l'épaulement 141 de calage des nervures.

Le mode de fonctionnement de ce troisième mode de réalisation préféré de l'invention est le suivant. L'utilisateur met à feu la charge pyrotechnique en agissant comme cela est décrit pour le premier mode de réalisation préféré de l'invention. Les gaz émis arrivent sur la partie conique 135 du piston 118 qui joue le rôle d'un déflecteur en favorisant la diffusion desdits gaz autour du réservoir 120 qui subit alors une légère surcompression externe qui compense la pression interne due à la déformation du piston en caoutchouc 118 qui amorce un déplacement dans le réservoir 120. L'obturateur 119 souple et déformable s'écrase et ouvre sa paroi étanche 121 par l'intermédiaire des tubes d'injection 122 poinçonnant ladite paroi 121. Le principe actif liquide 117 est alors expulsé par les canaux des tubes d'injection 122 jusqu'à ce que la partie centrale du piston 118 arrive au contact de l'obturateur 119.

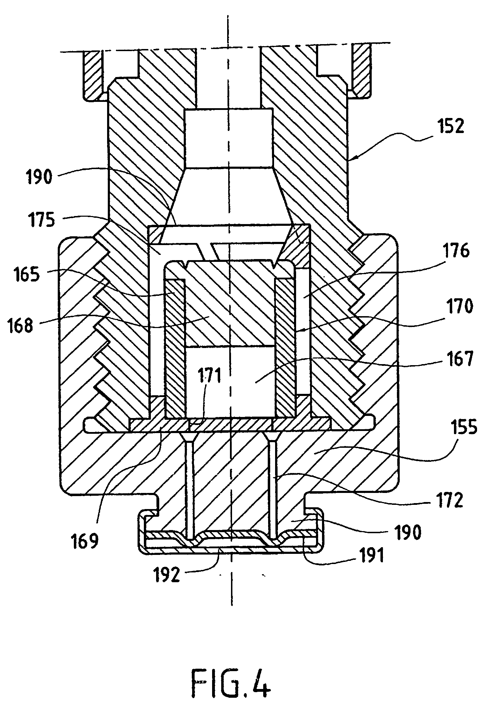

En se référant à la figure 4, un dispositif d'injection selon un quatrième mode de réalisation préféré de l'invention diffère du premier mode de réalisation sur les points décrits ci-après. Les gaz émis par le générateur non représenté sur la figure, sont produits suivant l'axe du corps 152 du dispositif d'injection, ledit corps 152 possédant un canal central présentant un évasement progressif 190 situé en amont du réservoir 170. Cet évasement 190 est compartimenté au moyen de trois nervures assurant le calage du réservoir tubulaire 170 et déterminant trois larges ouvertures 175 de façon à permettre l'écoulement des gaz autour du réservoir 170 dans l'espace de pressurisation 176. L'obturateur aval 169 est réalisé en caoutchouc et a la forme d'un cylindre creux de faible hauteur, fermé à l'une de ces deux extrémités par un disque dont le diamètre est supérieur au diamètre externe dudit cylindre. Ledit obturateur 169 est interposé entre le réservoir 170 de principe actif liquide 167 et la semelle 155 du corps 152, de façon à ce que, d'une part, le cylindre creux dudit obturateur 169 assure le calage et l'étanchéité entre la paroi latérale externe du tube 165 et la paroi latérale interne du corps 152 et, d'autre part, le disque dudit obturateur 169 reste au contact de la semelle 155 en ayant sa partie périphérique coincée de façon étanche entre le corps 152 et ladite semelle 155. La surface de la semelle 155 qui est au contact du disque de l'obturateur 169 présente à sa surface, quatre empreintes coniques au fond desquelles prennent naissance des canaux 172 d'injection traversant la semelle 155. En fait, la semelle 155 présente une avancée terminale 190 dans laquelle débouchent lesdits canaux 172. Le disque de l'obturateur 169 comporte quatre préperforations 171 au niveau des zones correspondant aux empreintes coniques de la semelle 155. L'avancée terminale 190 est obturée par une couche d'élastomère 191 et un capuchon métallique 192 serti autour de ladite avancée terminale 190. Le mode de fonctionnement de ce quatrième mode de réalisation préféré de l'invention est le suivant.

L'utilisateur retire la couche d'élastomère 191 et le capuchon métallique 192 servant de protection du dispositif d'injection. Il applique alors l'extrémité de l'avancée terminale 190 contre la peau du patient à traiter. Il met à feu ensuite la charge pyrotechnique en agissant comme cela est décrit pour le premier mode de réalisation préféré de l'invention.

Les gaz émis arrivent sur le piston 168 en même temps qu'ils envahissent l'espace de pressurisation 176 par les ouvertures 175 d'évacuation mettant instantanément le réservoir 170 en compression. Lorsque la pression est suffisante pour rompre sa collerette externe, le piston 168 amorce un déplacement dans le réservoir 170 créant une surpression interne qui ouvre l'obturateur aval 169 au niveau de ses quatre zones prépercées. Le principe actif liquide 167 est alors expulsé à très haute vitesse par les canaux 172 traversant la semelle 155 vers la peau du patient à traiter.

## Revendications

1. Dispositif d'injection cutanée comprenant un corps (2, 52, 102, 152) et destiné à injecter un principe actif (17, 117, 167) liquide contenu dans un réservoir tubulaire (20, 70, 120, 170) constitué par un tube (15, 65, 115, 165) fermé à son extrémité amont par un piston (18, 118, 168) de refoulement et comportant à son extrémité aval un ensemble d'injection, ce réservoir étant logé dans le corps (2, 52, 102, 152) du dispositif d'injection qui comporte en amont du piston (18, 118, 168) un générateur de gaz (4, 104) à haute pression permettant d'exercer une pression sur ce piston (18, 118, 168) de refoulement, **caractérisé en ce qu'**un espace de pressurisation (26, 76, 126, 176) par les gaz du générateur (4, 104) est ménagé entre le réservoir tubulaire (20, 70, 120, 170) et la paroi interne du corps (2, 52, 102, 152).

2. Dispositif selon la revendication 1, **caractérisé en ce que** la paroi interne du corps (102) comporte au moins trois nervures longitudinales (140) de centrage du réservoir tubulaire (120).

3. Dispositif selon la revendication 1, **caractérisé en ce que** le tube (15, 65, 115, 165) est réalisé en verre.

4. Dispositif selon la revendication 1, **caractérisé en ce que** la surface externe du piston (118) de refoulement, dirigée vers le générateur de gaz (104) constitue un déflecteur (135) des gaz vers l'espace de pressurisation(126).

5. Dispositif selon la revendication 2, **caractérisé en ce que** le piston (118, 168) de refoulement s'étend au dessus du tube (115, 165).

6. Dispositif selon la revendication 5, **caractérisé en ce que** le piston (18, 118, 168) comporte une amorce de rupture par cisaillement qui correspond aux dimensions internes du tube (15, 65, 115, 165).

7. Dispositif selon l'une quelconque des revendications 1, 4 ou 5, **caractérisé en ce que** le générateur de gaz (4, 104) est un générateur pyrotechnique.

8. Dispositif selon la revendication 1, **caractérisé en ce que** l'ensemble d'injection comprend un obturateur aval (19, 79, 119) qui comporte au moins un trou borgne ménageant une paroi étanche crevable (21, 121), cette paroi s'étendant entre le fond de ce trou borgne et le principe actif (17, 117).

9. Dispositif selon la revendication 8, **caractérisé en ce que** l'obturateur aval (19, 79, 119, 169) s'étend en dessous du tube (15, 65, 115, 165) et se trouve encastré dans le corps (2, 52, 102, 152).

10. Dispositif selon la revendication 9, **caractérisé en ce que** le corps (52) comprend une semelle (55) et un organe de compression (82) qui est interposé entre l'obturateur aval (79) et ladite semelle (55).

11. Dispositif selon l'une quelconque des revendications 8 ou 9, **caractérisé en ce que** le piston (18, 118, 168) de refoulement et l'obturateur aval (19, 79, 119, 169) sont au moins partiellement réalisés en matériau amortissant.

12. Dispositif selon l'une quelconque des revendications 1 ou 7, **caractérisé en ce que** le générateur de gaz (4) comporte des orifices d'évacuation (25, 175) des gaz dirigés au moins partiellement vers l'espace de pressurisation (26, 176).

13. Dispositif selon la revendication 9, **caractérisé en ce que** l'obturateur aval (169) comporte au moins une préperforation (171) en correspondance avec un canal (172) d'injection.

14. Procédé de fabrication d'un dispositif d'injection cutanée conforme à l'une quelconque des revendications précédentes, **caractérisé en ce que** :
- dans une première étape, l'ensemble constitué par le réservoir tubulaire (20, 70, 120) rempli du principe actif (17, 117) et encastré dans la semelle (5, 55, 105) munie d'au moins un tube d'injection (22, 122), est protégé par un embout amovible étanche (24), l'ensemble des opérations de cette première étape étant réalisé dans les conditions pharmaceutiques d'hygiène et de sécurité exigées pour la fabrication de médicaments,
- dans une deuxième étape, cet ensemble est fixé de façon étanche à l'extrémité aval de la partie centrale tubulaire (3) du corps (2, 52, 102) dont l'autre extrémité reçoit le générateur de gaz (4, 104) à haute pression, l'ensemble de ces opérations d'assemblage étant réalisé dans des conditions simples d'hygiène et de sécurité.

## Patentansprüche

1. Haut-Injektionsvorrichtung mit einem Körper (2, 52, 102, 152), die dazu dient, einen flüssigen Wirkstoff (17, 117, 167) zu injizieren, der in einem rohrförmigen Behälter (20, 70, 120, 170) enthalten ist, der durch ein Rohr (15, 65, 115, 165) gebildet wird, das an seinem oberen Ende durch einen Verdrängungskolben (18, 118, 168) verschlossen ist und an seinem unteren Ende eine Injektionseinheit aufweist, wobei dieser Behälter im Körper (2, 52, 102, 152) der Injektionsvorrichtung angeordnet ist, der oberhalb des Kolbens (18, 118, 168) einen Hochdruck-Gasgenerator (4, 104) aufweist, der es ermöglicht, Druck auf den Verdrängungskolben (18, 118, 168) auszuüben,
**dadurch gekennzeichnet, dass** ein Druckraum (26, 76, 126, 176) der durch die Gase des Generators (4, 104) unter Druck gesetzt werden kann, zwischen dem rohrförmigen Behälter (20, 70, 120, 170) und der Innenwand des Körpers (2, 52, 102, 152) ausgebildet ist.

2. Vorrichtung nach Anspruch 1, **dadurch gekennzeichnet, dass** die Innenwand des Körpers (102) mindestens drei Längsrippen (140) zur Zentrierung des rohrförmigen Behälters (120) aufweist.

3. Vorrichtung nach Anspruch 1, **dadurch gekennzeichnet, dass** das Rohr (15, 65, 115, 165) aus Glas besteht.

4. Vorrichtung nach Anspruch 1, **dadurch gekennzeichnet, dass** die zum Gasgenerator (104) hin gerichtete Außenfläche des Kolbens (118) einen Deflektor (135) der Gase zum Druckraum (126) hin bildet.

5. Vorrichtung nach Anspruch 2, **dadurch gekennzeichnet, dass** sich der Verdrängungskolben (118, 168) über das Rohr (115, 165) erstreckt.

6. Vorrichtung nach Anspruch 5, **dadurch gekennzeichnet, dass** der Kolben (18, 118, 168) eine auf Scherung ansprechende Sollbruchstelle aufweist, die den Innenabmessungen des Rohrs (15, 65, 115, 165) entspricht.

7. Vorrichtung nach einem der Ansprüche 1, 4 oder 5, **dadurch gekennzeichnet, dass** der Gasgenerator (4, 104) ein pyrotechnischer Generator ist.

8. Vorrichtung nach Anspruch 1, **dadurch gekennzeichnet, dass** die Injektionseinheit einen unterseitigen Verschluss (19, 79, 119) aufweist, der mindestens ein Sackloch aufweist, das eine dichte durchbrechbare Wand (21, 121) bildet, wobei sich diese Wand zwischen dem Boden dieses Sacklochs und dem Wirkstoff (17, 117) erstreckt.

9. Vorrichtung nach Anspruch 8, **dadurch gekennzeichnet, dass** sich der unterseitige Verschluss (19, 79, 119, 169) unterhalb des Rohrs (15, 65, 115, 165) erstreckt und im Körper (2, 52, 102, 152) eingesetzt ist.

10. Vorrichtung nach Anspruch 9, **dadurch gekennzeichnet, dass** der Körper (52) ein Fußteil (55) und ein zusammendrückbares Element (82) aufweist, das zwischen dem unterseitigen Verschluss (79) und dem Fußteil (55) angeordnet ist.

11. Vorrichtung nach Anspruch 8 oder 9, **dadurch gekennzeichnet, dass** der Verdrängungskolben (18, 118, 168) und der unterseitige Verschluss (19, 79, 119, 169) mindestens teilweise aus einem dämpfenden Material besteht.

12. Vorrichtung nach einem der Ansprüche 1 oder 7, **dadurch gekennzeichnet, dass** der Gasgenerator (4) Ausströmöffnungen (25, 175) für die Gase aufweist, die mindestens teilweise zum Druckraum (26, 176) gerichtet sind.

13. Vorrichtung nach Anspruch 9, **dadurch gekennzeichnet, dass** der unterseitige Verschluss (169) mindestens eine Vorperforation (171) aufweist; die einem Injektionskanal (172) zugeordnet ist.

14. Verfahren zur Herstellung einer Haut-Injektionsvorrichtung nach einem der vorhergehenden Ansprüche, **dadurch gekennzeichnet, dass**
- in einem ersten Schritt die Einheit, die durch den mit dem Wirkstoff (17, 117) gefüllten, rohrförmigen Behälter (20, 70, 120) gebildet wird und in dem mit mindestens einem Injektionsrohr (22, 122) versehenen Fußteil (5, 55, 105) eingesetzt ist, durch eine lösbare dichte Abdeckung (24) geschützt wird, wobei sämtliche Vorgänge dieses ersten Schrittes unter den für die Herstellung von Medikamenten erforderlichen pharmazeutischen Hygiene- und Sicherheitsbedingungen stattfinden,
- in einem zweiten Schritt diese Einheit dicht am unteren Ende des mittleren, rohrförmigen Teils (3) des Körpers (2, 52, 102) befestigt wird, dessen anderes Ende den Hochdruck-Gasgenerator (4, 104) aufnimmt, wobei sämtliche Zusammenbauvorgänge unter einfachen Hygiene- und Sicherheitsbedingungen stattfinden.

## Claims

1. Cutaneous injection device comprising a body (2, 52, 102, 152) and intended for the injection of a liquid active principle (17, 117, 167) contained in a tubular reservoir (20, 70, 120, 170) consisting of a tube (15, 65, 115, 165) closed at its upstream end by a delivery piston (18, 118, 168) and having at its downstream end an injection assembly, this reservoir being housed in the body (2, 52, 102, 152) of the injection device which includes upstream to the piston (18, 118, 168), a high pressure gas generator (4, 104) making it possible to exert a pressure on this delivery piston (18, 118, 168), **characterised in that** a space (26, 76, 126, 176) for pressurisation by gases from the generator (4, 104) is provided between the tubular reservoir (20, 70, 120, 170) and the internal wall of the body (2, 52, 102, 152).

2. Device according to claim 1 **characterised in that** the internal wall of the body (102) has at least three longitudinal ribs (140) for centring the tubular reservoir (120).

3. Device according to claim 1, **characterised in that** the tube (15, 65, 115, 165) is made of glass.

4. Device according to claim 1, **characterised in that** the external surface of the delivery piston (118), directed towards the gas generator (104) constitutes a deflector (135) for gases towards the pressurizing space (126).

5. Device according to claim 2, **characterised in that** the delivery piston (118, 168) extends above the tube (115, 165).

6. Device according to claim 5, **characterised in that** the piston (18, 118, 168) includes a detonator breaking by shear which corresponds to the internal dimensions of the tube (15, 65, 115, 165).

7. Device according to any one of claims 1, 4 or 5, **characterised in that** the gas generator (4, 104) is a pyrotechnic generator.

8. Device according to claim 1, **characterised in that** the injection assembly includes a downstream stopper (19, 79, 119) which has at least one blind hole providing a burstable leakproof wall (21, 121), this wall extending between the bottom of this blind hole and the active principle (17, 117).

9. Device according to claim 8, **characterised in that** the downstream stopper (19, 79, 119, 169) extends below the tube (15, 65, 115, 165) and is situated recessed in the body (2, 52, 105, 152).

10. Device according to claim 9, **characterised in that** the body (52) includes a base (55) and a compression device (82) which is interposed between the downstream stopper (79) and the said base (55).

11. Device according to either of claims 8 or 9, **characterised in that** the delivery piston (18, 118, 168) and the downstream stopper (19, 79, 119, 169) are at least partially made of a cushioning material.

12. Device according to either of claims 1 or 7, **characterised in that** the gas generator (4) has orifices (25, 175) for evacuating the gases directed at least partially towards the pressurisation space (26, 176).

13. Device according to claim 9, **characterised in that** the downstream stopper (169) has at least one pre-perforation (171) corresponding with an injection channel (172).

14. Process for manufacturing a cutaneous injection device according to any one of the preceding claims, **characterised in that** :
- in a first step, the assembly consisting of the tubular reservoir (20, 70, 120) filled with the active principle (17, 117) and recessed into the base (5, 55, 105) provided with at least one injection tube (22, 122), is protected by a leakproof detachable end piece (24), all the operations of this first step being carried out under hygienic and safe pharmaceutical conditions required for the manufacture of medications,
- in a second step, this assembly is fixed in a leakproof manner to the downstream end of the central tubular part (3) of the body (2, 52, 102) of which the other end receives the high pressure gas generator (4, 104), all these assembly operations being carried out under simple hygienic and safe conditions.
